(19) Europäisches Patentamt
European Patent Office
Office européen des brevets

(11) Veröffentlichungsnummer: **0 406 248 B1**

# (12) EUROPÄISCHE PATENTSCHRIFT

(45) Veröffentlichungstag der Patentschrift: **08.06.94**

(51) Int. Cl.$^5$: **A61K 31/80**

(21) Anmeldenummer: **89901945.9**

(22) Anmeldetag: **19.01.89**

(86) Internationale Anmeldenummer:
**PCT/EP89/00058**

(87) Internationale Veröffentlichungsnummer:
**WO 90/07930 (26.07.90 90/17)**

Die Akte enthält technische Angaben, die nach dem Eingang der Anmeldung eingereicht wurden und die nicht in dieser Patentschrift enthalten sind.

## (54) VERWENDUNG VON DIMETHYLPOLYSILOXAN ZUR BEHANDLUNG VON ERKRANKUNGEN DES GASTROINTESTINALTRAKTES.

(43) Veröffentlichungstag der Anmeldung:
**09.01.91 Patentblatt 91/02**

(45) Bekanntmachung des Hinweises auf die Patenterteilung:
**08.06.94 Patentblatt 94/23**

(84) Benannte Vertragsstaaten:
**AT BE CH DE FR GB IT LI LU NL SE**

(56) Entgegenhaltungen:
**FR-M- 6 885**
**GB-A- 1 097 955**
**GB-A- 2 033 915**
**US-A- 2 951 011**

**Dictionnaire VIDAL, 1986, O.V.P. (Paris,FR), page 1361, SILIGAZ**

(73) Patentinhaber: **Schmidt, Alfred**
**Leinpfad 2**
**D-22301 Hamburg(DE)**

Patentinhaber: **Upmeyer, Jürgen**
**Mauerkircherstrasse 197**
**D-81925 München(DE)**

(72) Erfinder: **Schmidt, Alfred**
**Leinpfad 2**
**D-22301 Hamburg(DE)**
Erfinder: **Upmeyer, Jürgen**
**Mauerkircherstrasse 197**
**D-81925 München(DE)**

(74) Vertreter: **VOSSIUS & PARTNER**
**Postfach 86 07 67**
**D-81634 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 406 248 B1

## Beschreibung

Die Erfindung Liegt auf dem Gebiete der medikamentösen Behandlung von Erkrankungen des Ösophagus und Gastrointestinaltraktes.

Der entschäumende Effekt von Dimethylpolysiloxan ist gut untersucht und wird bereits als Antiflatulenz oder in der Sonografievorbereitung therapeutisch genutzt.

In Dictionnaire Vidal, 1986, S. 1361 "Siligaz", wird ein Dimethylpolysiloxan-Präparat beschrieben, das für die Indikation Sodbrennen verwendet werden kann.

In der FR-M 6885 wird angegeben, daß Organopolysiloxane zur Behandlung von Gastritis verwendet werden.

GB-A2 2,033,913 beschreibt ein Antazida-Mittel in Form einer Kautablette, das ein Gemisch aus einem Antazida, Dimethylpolysiloxan und Kieselgel umfaßt.

In der Roten Liste 1988, Nr. 59287 bis 59292 werden Dimethylpolysiloxan-haltige Präparate für die Indikationen Meteorismus, Völlegefühl und Flatulenz beschrieben.

Es wurde überraschenderweise gefunden, daß Dimethylpolysiloxan mit besonders vorteilhafter Wirkung zur Behandlung von entzündlichen und ulcerösen Erkrankungen des Gastrointestinaltraktes eingesetzt werden kann. Der entschäumende Effekt von Dimethylpolysiloxan ist korreliert mit den weiteren physikochemischen Eigenschaften Filmbildung und wandständige Adhäsion. Es hat sich überraschenderweise gezeigt, daß Dimethylpolysiloxan auch über längere Zeiträume ohne nachteilige Auswirkungen oral verabreicht werden kann, um im Ösophagus sowie im Gastrointestinaltrakt des Menschen eine schützende Schicht zu bilden und aufrecht zu erhalten, die den Heilungsprozeß beim Vorliegen von Erkrankungen der beschriebenen Art unterstützt.

Die Erfindung betrifft somit die Verwendung von Dimethylpolysiloxan ohne Zusatz von Antazida zur Herstellung eines Arzneimittels zur Behandlung von ulcerösen Erkrankungen des Magens und Duodenums (Ulcus ventriculi und Ulcus duodeni), entzündliche Erkrankungen des Ösophagus und der Antrumgastritis.

Die erfindungsgemäße Verwendung von Dimethylpolysiloxan erfährt dadurch eine Steigerung ihrer Wirksamkeit, wenn das Mittel in Verbindung mit Kieselgel eingenommen wird.

Der mit dem Mittel im Ösophagus und Gastrointestinaltrakt erzeugte Überzug wird hierdurch gelartig ausgebildet. Diese Schicht kommt dem physiologischen Schleim (z. B. Magenschleim) sehr nahe, der den Schutz der Schleimhäute von Ösophagus und Gastrointestinaltrakt bewirkt.

Mit dem erfindungsgemäß eingesetzten Mittel gelingt es gezielt, die Schleimhäute von Ösophagus und Gastrointestinaltrakt über eine längere Zeitspanne, durch entsprechende Einnahme auch über Tage, Wochen oder Monate zu beschichten und damit gegen die aggressiven Inhaltsstoffe des Magen-Darmtraktes, wie Salzsäure und Verdauungsenzyme, zu schützen. Der mit dem erfindungsgemäß eingesetzten Mittel herbeigeführte Schutz bewirkt einmal eine Einschränkung der Aktivität gewebezerstörender Mechanismen und zum anderen eine vergleichsweise ungestörte Regeneration der Wandungen bei der Zellneubildung sowie der Geschwürreinigung. Das erfindungsgemäß eingesetzte Mittel bewirkt zusätzlich eine Anhebung des pH-Wertes des Magens.

Das subjektive Empfinden von Patienten verbessert sich durch die Einnahme des Mittels, das 'Sodbrennen' der häufig hyperaciden Patienten verschwindet.

<u>Untersuchungen</u>

Untersucht wurden in Abhängigkeit von Dosis und Zeit:

1. Verteilung und Filmbildung von Dimethylpolysiloxan im Magen
2. Verteilung und Filmbildung von Dimethylpolysiloxan im Duodenum

Als freiwillige Probanden wurden 8 gesunde Männer im Alter von 25 und 40 Jahren herangezogen (Tabelle 1). Die Studie wurde als kontrollierte, randomisierte Phase-I-Studie im Cross-over-Design durchgeführt. Für das Cross-over-Design wurden die 8 Probanden randomisiert und den beiden Dosierungsformen zugeteilt. Die Untersuchungen wurden in einem Abstand von 7 Tagen durchgeführt.

Es wurden zwei Dosierungen pro Proband im Cross-over-Design angewandt:

Dosierung 1: 80 mg Dimethylpolysiloxan: (2 Dimethylpolysiloxan-Kautabletten zu 40 mg)
Dosierung 2: 160 mg Dimethylpolysiloxan: (4 Dimethylpolysiloxan-Kautabletten zu 40 mg)

Die Zusammensetzung der Dimethylpolysiloxan-Kautabletten ergibt sich aus Tabelle 8.

Unmittelbar vor und während der Studie durften keine weiteren Medikamente eingenommen werden. Am Tag der Untersuchung durfte darüber hinaus nicht geraucht werden.

## Kontrolluntersuchungen

Zur Eingangsuntersuchung wurde eine umfangreiche Anamnese erhoben; dazu die internistischen und neurologischen Basisuntersuchungen. Außerdem wurde ein Laborstatus erhoben.

An beiden Untersuchungstagen wurden für alle Probanden folgende gastroenterologischen Verlaufsuntersuchungen durchgeführt:

1. Gastroskopie mit Dokumentation von Ösophagus, Magen und Duodenum unmittelbar vor Zerkauen der Dimethylpolysiloxan-Tabletten.
2. Die gleichen Untersuchungen inklusive der Dokumentation 30 Minuten nach Einnahme der Kautabletten.
3. Die gleichen Untersuchungen inklusive der Dokumentation 2 Stunden nach Kauen der Dimethylpolysiloxan-Kautabletten.

Die Untersuchungen wurden pro Proband zweimal, jeweils mit der alternativen Dosierung, im Abstand von 7 Tagen durchgeführt. Bei einer zweiten Verlaufsuntersuchung wurden bei jeder Gastroskopie zusätzlich eine pH-Metrie des Mageninhaltes vorgenommen.

Die Probanden durften vor der jeweiligen Medikamenteneinnahme und Gastroskopie mindestens 6 Stunden keine Nahrungsmittel und keine Flüssigkeit zu sich genommen haben.

Zur Endoskopie-Vorbereitung erhielt jeder Proband 0,5 mg Atropin in 1 ml NACL-Lösung subkutan appliziert. Als Lokalanästhetikum für den Mund und Rachenraum wurde Novescine Wander 1 % Lösung (Oxybuprocain-HCL) gegeben.

Die gastroskopischen Untersuchungen wurden mit einem Gerät Olympus Typ $P_{10}$ durchgeführt. Die Dokumentation erfolgte durch visuellen Befund und Foto.

## Ergebnisse

### 1. Veränderung der pH-Werte des Magens

Tabelle 2 gibt eine Übersicht über die pH-Wert-Veränderungen, und zwar bezogen auf den pH-Wert der Untersuchung unmittelbar vcr Medikation und 2 Stunden nach Medikation.

Die Dimethylpolysiloxan-Gabe führt zu einer deutlichen Erhöhung des pH-Wertes, d. h. einer deutlichen Verschiebung vom stark sauren in Richtung des alkalischen Bereichs, und zwar unabhängig von der Anzahl der verabreichten Kautabletten. Trotz der geringen Probandenzahl ist dieser Unterschied auf dem 5 % Niveau signifikant.

Proband 7 wurde aus der Berechnung herausgenommen, weil er einen starken Narbenbulbus hatte und darüber hinaus der pH-Wert bereits nahe am alkalischen Bereich lag (pH-Wert 6,05 bzw. 6,83).

### 2. Filmbildung

Tabelle 3 gibt summarisch die Einzelergebnisse der Tabellen 4 bis 7 wieder. Ziel der Studie war es, ein Verweilen des Dimethylpolysiloxans nach Applikation von Kautabletten im Magen bzw. Duodenum in dem beobachteten Zeitraum von 2 Stunden zu belegen. Die Ergebnisse sind eindeutig. Bereits eine Gabe von 2 Tabletten Dimethylpolysiloxan (80 mg) führt bei allen Probanden zu einer Reaktion. Diese wird durch die Gabe von 4 Tabletten Dimethylpolysiloxan (160 mg) noch deutlicher.

Bei 360 mg zeigte sich auf der Magenschleimhaut bei 7 von 8 Probanden relativ viel milchiges Sekret als Beobachtungsparameter.

Die Einzelbefunde (bzw. die zusammenfassenden Befunde) sind in den Tabellen 4 bis 7 festgehalten.

Die Auswertung der visuellen Befundung und der Fotodokumentation läßt die zusammenfassende Beurteilung zu, daß Dimethylpolysiloxan-Kautabletten bzw. Dimethylpolysiloxan in der Lage sind, einen adhäsiven Film auf der Magenschleimhaut sowie im proximalen Teil des Duodenums zu bilden.

Tab. 1

| Anamnestische Daten der Probanden der Dimethylpolysiloxan-Studie | | | | | | |
|---|---|---|---|---|---|---|
| | Mittelwert | SD | Min | Max | Median | |
| Alter | 32,15 | 4,51 | 25 | 40 | 31,5 | Jahre |
| Größe | 179,75 | 7,31 | 170 | 191 | 181 | cm |
| Gewicht | 78 | 7,68 | 63 | 90 | 77 | kg |
| systol. Blutdruck | 116 | 9,99 | 101 | 130 | 114,5 | mmHg |
| diast. Blutdruck | 78,62 | 8,44 | 62 | 90 | 81 | mmHg |
| Puls | 63,5 | 10,94 | 53 | 88 | 60,5 | /Min |

Tabelle 8

| Dimethylpolysiloxan-Kautablette 40 mg | | | |
|---|---|---|---|
| Zusammensetzung pro Tablette: | | | |
| I. | 1) | Dimethylpolysiloxan DAB9 | 40,000 mg |
| | 2) | Hochdisperses Siliciumdioxid DAB9 | 12,555 mg |
| | 3) | Pfefferminzöl DAB9 | 0,300 mg |
| | | Anisöl DAB9 | 0,033 mg |
| | 4) | Mikrokristalline Cellulose DAB9 | 200,000 mg |
| II. | 5) | Mannitol DAB9 | 338,111 mg |
| | 6) | Saccharin-Natrium DAB9 | 0,667 mg |
| | 7) | Carboxymethylcellulose-Natrium DAB9 | 1,667 mg |
| III. | 9) | Magnesiumstearat DAB9 | 3,333 mg |
| | 10) | Maisstärke DAB9 | 3,333 mg |

Tab. 2: Veraenderung der pH-Werte des Magens bei Medikation mit Dimethylpolysiloxan

| Prob. nr | 1 | 2 | 3 | 4 | 5 | 6 | 7 | 8 |
|---|---|---|---|---|---|---|---|---|
| Vor Med. | 1,80 | 1,96 | 1,92 | 1,98 | 1,51 | 2,11 | 6,03 | 1,69 |
| 30 Min. nach Med. | 2,06 | - | - | - | - | - | - | - |
| 2 Std. nach Med. | 2,15 | 2,04 | 4,48 | 7,49 | 3,84 | 2,32 | 6,83 | 2,67 |
| Anz. Tab. | 2 | 4 | 4 | 2 | 2 | 2 | 4 | 2 |

Eine positive Beeinflussung des pH-Wertes durch die Medikation wird durch den Vorzeichentest ($p < 0,05$, zweiseitig) bestaetigt. Ein Einfluss der Anzahl der Tabletten auf die Erhoehung des pH-Wertes kann man jedoch nicht feststellen.

Proband 7 wies einen Narben bulbus ohne Stenose auf wurde aufgrund der hohen pH-Werte nicht mit in die Berechnung einbezogen.

Tab.3: Positive Reaktion bei Gabe von Dimethylpolysiloxan-Kautabletten

2 Tabletten Dimethylpolysiloxan

| Probanden mit positiver Reaktion* | keiner Reaktion | n |
|---|---|---|
| 8 | 0 | 8 |

(* Die Reaktion war bei 5 von 8 Probanden minimal milchiges Sekret)

4 Tabletten Dimethylpolysiloxan

| Probanden mit positiver Reaktion** | keiner Reaktion | n |
|---|---|---|
| 8 | 0 | 8 |

(** Die Reaktion war bei 1 von 8 Probanden minimal milchiges Sekret, ansonsten immer relativ viel milchiges Sekret)

**Tabelle 4**

Befunde der ersten Untersuchung

| | Zeit-Punk* | Anz. Tabl. | Befund |
|---|---|---|---|
| Proband Nr. 1 | 1 | 4 | Zeichen einer Antrumgastritis. Sonst Duodenum, Magen und Ösophagus makroskopisch unauffällig. |
| Proband Nr. 1 | 2 | 4 | Im Fundus Magensaft; milchartig, trübe und flokkig. |
| Proband Nr. 1 | 3 | 4 | Nur noch minimale Reste im Fundus. Sonst bis zum tiefen Duodenum frei. |
| Proband Nr. 2 | 1 | 2 | Zeichen einer leichten Antrumgastritis. Sonst Duodenum, Magen und Ösophagus makroskopisch unauffällig. |
| Proband Nr. 2 | 2 | 2 | Im Fundus Magensaft; milchartig, trübe und flokkig |
| Proband Nr. 2 | 3 | 2 | Noch milchige Substanz im Fundus. Duodenum und Antrum frei. |

* Zeitpunkt 1 = unmittelbar vor Medikation
Zeitpunkt 2 = 1/2 Stunde nach Medikation
Zeitpunkt 3 = 2 Stunde nach Medikation

Tabelle 4, Fortsetzung

| | Zeit-Punkt | Anz. Tabl. | Befund |
|---|---|---|---|
| Proband Nr. 3 | 1 | 2 | Kleine Hiatushernie mit Reflux 0-1 Grades. Sonst Duodenum, Magen und Ösophagus makroskopische unauffällig. |
| Proband Nr. 3 | 2 | 2 | Im Fundus Magensaft; milchartig, trübe und flokkig |
| Proband Nr. 3 | 3 | 2 | Winzige Reste von milchig, flockigen Auflagerungen. Fundus flächig, filmartig belegt. Duodenum frei. |
| Proband Nr. 4 | 1 | 4 | Duodenum, Magen und Ösophagus makroskopisch unauffällig. |
| Proband Nr. 4 | 2 | 4 | Im Fundus Magensaft; milchartig, trübe und flokkig |
| Proband Nr. 4 | 3 | 4 | Leicht trüber, milchiger Magensaft. Sonst Duodenum, Magen, Ösophagus unauffällig. |

Tabelle 4, Fortsetzung

| | Zeit-Punkt | Anz. Tabl. | Befund |
|---|---|---|---|
| Proband Nr. 5 | 1 | 4 | Kleine Hiatushernie mit Refluxösophagitis (Grad 0 - I). Sonst Duodenum, Magen und Ösophagus makroskopisch unauffällig. |
| Proband Nr. 5 | 2 | 4 | Antrum mit schorfig-milchigem Belag im Fundus. Stark trüb, milchige Flüssigkeit. |
| Proband Nr. 5 | 3 | 4 | Reste von milchartigen Flocken im Fundus. Vereinzelte Plaques im Antrum. Duodenum frei. |
| Proband Nr. 6 | 1 | 4 | Duodenum, Magen und Ösophagus unauffällig. |
| Proband Nr. 6 | 2 | 4 | Vereinzelte weißliche, milchartige Stippchen im Antrum. Sonst trübe, milchartige Belag im Fundus. |
| Proband Nr. 6 | 3 | 4 | Stark trübe, milchartige Flüssigkeit, dem Fundus aufgelagert Vereinzelte Plaques im Antrum. Duodenum frei. |

**Tabelle 4, Fortsetzung**

| | Zeit-Punkt | Anz. Tabl. | Befund |
|---|---|---|---|
| Proband Nr. 7 | 1 | 2 | Narben bulbus ohne Stenose. Magen und Ösophagus unauffällig. |
| Proband Nr. 7 | 2 | 2 | dünnflüssig, milchige Auflagerungen im Fundus. Antrum, Duodenum frei. |
| Proband Nr. 7 | 3 | 2 | Minimal fleckige, milchige Auflagerungen im Magen Fundus. Sonst Duodenum und Antrum frei. |
| Proband Nr. 8 | 1 | 2 | Duodenum, Magen und Ösophagus unauffällig. |
| Proband Nr. 8 | 2 | 2 | Dünnflüssiges, jedoch trübes, milchiges Sekret im Fundus, filmartiger Überzug. Antrum, Duodenum frei. |
| Proband Nr. 8 | 3 | 2 | Dünnflüssiges, milchiges Sekret als filmartiger Überzug. Sekret trübe. Antrum, Duodenum frei. |

Tabelle 5

Befunde der zweiten Untersuchung (nach 7 Tagen)

| | Zeit Punkt* | Anz. Tabl. | Befund |
|---|---|---|---|
| Proband Nr. 1 | 1 | 2 | Duodenum, Magen, Ösophagus unauffällig, Antrumgastritis makroskopisch deutlich gebessert. pH 1.80 |
| Proband Nr. 1 | 2 | 2 | Deutlich weniger, milchartiges Sekret und Filmüberzug. Sonst Befund unverändert. pH 2.06 |
| Proband Nr. 1 | 3 | 2 | Minimales milchartiges Sekret im Fundus. Duodenum, Magen unauffällig. pH 2.15 |
| Proband Nr. 2 | 1 | 4 | Weiterhin Zeichen einer leichten Antrumgastritis. Sonst Duodenum, Magen und Ösophagus unauffällig. pH 1.96 |
| Proband Nr. 2 | 2 | 4 | Sehr viel dickflüssig-milchiges Sekret im Fundus. Sonst Antrum frei |
| Proband Nr. 2 | 3 | 4 | sehr viel dickflüssig-milchiges Sekret im Fundus. Antrum, Duodenum frei. pH 2.04 |

* Zeitpunkt 1 = unmittelbar vor Medikation
Zeitpunkt 2 = 1/2 Stunde nach Medikation
Zeitpunkt 3 = 2 Stunde nach Medikation

**Tabelle 5, Fortsetzung**

| | Zeit-Punkt | Anz. Tabl. | Befund |
|---|---|---|---|
| Proband Nr. 3 | 1 | 4 | Kleine Hiatushernie mit Reflux 0-1 Grades. Sonst Duodenum, Magen und Ösophagus makroskopisch unauffälig. pH 1.92 |
| Proband Nr. 3 | 2 | 4 | Viel dickflüssig-milchiges Sekret im Antrum und Fundus, Duodenum frei. |
| Proband Nr. 3 | 3 | 4 | Sehr viel Reste von milchigem Überzug und Sekret im Antrum und Fundus. Duodenum frei. pH 4.48 |
| Proband Nr. 4 | 1 | 2 | Duodenum, Magen und Ösophagus makroskopisch unauffällig. pH 1.98 |
| Proband Nr. 4 | 2 | 2 | Minimal milchiges Sekret im Fundus. Duodenum, Antrum frei. |
| Proband Nr. 4 | 3 | 2 | Nur noch Reste von trübem, milchigem Sekret im Fundus. Duodenum und Antrum sonst frei. pH 7.49 |

Tabelle 5, Fortsetzung

| | Zeit-Punkt | Anz. Tabl. | Befund |
|---|---|---|---|
| Proband Nr. 5 | 1 | 2 | Kleine Hiatusherie mit Refluxösophagitis (Grad 0 - 1).<br>pH 1.51 |
| Proband Nr. 5 | 2 | 2 | Minimal milchiges Sekret im Corpus und im Antrum. Duodenum frei. |
| Proband Nr. 5 | 3 | 2 | Wenig trübes Sekret im Fundus. Antrum, Duodenum frei.<br>pH 3.84 |
| Proband Nr. 6 | 1 | 2 | Duodenum, Magen und Ösophagus unauffällig.<br>pH 2.11 |
| Proband Nr. 6 | 2 | 2 | Wenig milchiges, trübes Sekret im Fundus und Corpus. |
| Proband Nr. 6 | 3 | 2 | Minimales Sekret im Duodenum und im Magen.<br>pH 2.32 |

**Tabelle 5, Fortsetzung**

| | Zeit-Punkt | Anz. Tabl. | Befund |
|---|---|---|---|
| Proband Nr. 7 | 1 | 4 | Narben bulbus ohne Stenose. Magen und Ösophagus unauffällig. pH 6.03 |
| Proband Nr. 7 | 2 | 4 | Wenig milchig trübes Sekret im Fundus. Antrum Duodenum frei. |
| Proband Nr. 7 | 3 | 4 | Minimales Sekret im Fundus ohne die milchartige Trübung. Duodenum, Antrum frei. pH 6.83 |
| Proband Nr. 8 | 1 | 4 | Duodenum, Magen und Ösophagus unauffällig. pH 1.69 |
| Proband Nr. 8 | 2 | 4 | Minimales, milchartiges Sekret als filmartiger Überzug. Tabletten unzerkaut im Corpus. Duodenum frei. |
| Proband Nr. 8 | 3 | 4 | Jetzt sehr viel trübes, milchartiges Sekret als filmartiger Überzug im Fundus. Corpus, Duodenum, Antrum frei. pH 2.67 |

Tab. 6

| Uebersicht der Befunde der Dimethylpolysiloxan-Studie | | | | | | |
|---|---|---|---|---|---|---|
| | Anz. Prob. | Haeufigkeit des Vorkommens | | | | |
| | | 2 T. | 4 T. | Z 2 | Z 3 | Z 2+3 |
| Zeichen einer Antrumgastritis | 2 | | | | | |
| Antrumgastritis makroskop. gebessert | 1 | | | | | |
| Duodenum und Antrum frei | 6 | 6 | 4 | | 4 | 6 |
| Duodenum frei | 3 | | 3 | 1 | 2 | |
| Corpus frei | 1 | | 1 | | 1 | |
| Minimales/wenig milchartiges Sekret | 6 | 5 | 1 | 2 | 2 | 2 |
| Minimale/Reste von fleckigen milchige Auflagerungen | 4 | 2 | 2 | 1 | 3 | |
| Vereinzelte Plaques im Antrum | 2 | 0 | 2 | 0 | 2 | |
| Milchartiger,trueber, flockiger Magensaft | 4 | 2 | 2 | 2 | | 2 |
| Milchige Auflagerungen | 5 | 3 | 2 | 2 | 1 | 2 |
| Sehr viel dickfluessigmilchiges Sekret | 3 | 0 | 3 | 1 | 1 | 1 |
| Sehr viel Reste von milchigem Ueberzug und Sekret | 1 | 0 | 1 | | 1 | |
| Abkuerzungen: Anz. Prob. = Anzahl der Probanden; T = Tabletten; Z = Zeitpunkt | | | | | | |

Tab. 7:

Dimethylpolysiloxan-Studie

| | Prob. nummer | Datum | Anz. Tabl. | Untersuchungs-zeitpunkt |
|---|---|---|---|---|
| Zeichen einer Antrum-gastritis | 1 | 15.3. | | 1 |
| Antrumgastritis makroskop. gebessert | 1 | 22.3. | | 1 |
| Zeichen einer Antrum-gastritis | 2 | 15.3. | | 1 |
| | 2 | 22.3. | | 1 |
| Kleine Hiatushernie | 3 | 15.3. | | 1 |
| | 3 | 22.3. | | 1 |
| | 5 | 15.3. | | 1 |
| | 5 | 22.3. | | 1 |
| Narben bulbus ohne Stenose | 7 | 15.3. | | 1 |
| | 7 | 22.3. | | 1 |
| Duodenum und Antrum frei | 2 | 15.3. | 2 | 3 |
| | 3 | 15.3. | 2 | 3 |
| | 4 | 22.3. | 2 | 2 und 3 |
| | 5 | 22.3. | 2 | 2 und 3 |
| | 7 | 15.3. | 2 | 2 und 3 |
| | 8 | 15.3. | 2 | 2 und 3 |
| | 2 | 22.3. | 4 | 2 und 3 |
| | 4 | 15.3. | 4 | 3 |
| | 7 | 22.3. | 4 | 2 und 3 |
| | 8 | 22.3. | 4 | 3 |
| Duodenum frei | 5 | 15.3. | 4 | 3 |
| | 6 | 15.3. | 4 | 3 |
| | 8 | 22.3. | 4 | 2 |
| Corpus frei | 8 | 22.3. | 4 | 3 |

Forts. Tab.7:

| | Prob. nummer | Datum | Anz. Tabl. | Untersuchungs-zeitpunkt |
|---|---|---|---|---|
| Minimales milchiges Sekret (Tabletten unzerkaut im Corpus) | 8 | 22.3. | 4 | 2 |
| Minimales milchartiges Sekret im Fundus | 1 | 22.3. | 2 | 2 und 3 |
| | 4 | 22.3. | 2 | 2 und 3 |
| | 5 | 22.3. | 2 | 3 |
| | 6 | 22.3. | 2 | 2 |
| Minimales milchiges Sekret im Corpus und Antrum | 5 | 22.3. | 2 | 2 |
| | 6 | 22.3. | 2 | 2 |
| Fundus mit minimalen fleckigen, milchigen Auflagerungen | 7 | 15.3. | 2 | 3 |
| | 1 | 15.3. | 4 | 3 |
| Wenig milchiges Sekret im Fundus | 7 | 22.3. | 4 | 2 |
| Magen mit minimalen fleckigen, milchigen Auflagerungen | 7 | 15.3. | 2 | 3 |
| Minimales Sekret im Duodenum und Antrum | 6 | 22.3. | 2 | 3 |
| Minimales Sekret im Fundus ohne milchartige Truebung | 7 | 22.3. | 4 | 3 |
| Vereinzelte weissliche Stippchen im Antrum | 6 | 15.3. | 4 | 2 |
| Winzige Reste von milchig, flockigen Auflagerungen | 3 | 15.3. | 2 | 3 |
| Reste von milchartigen Flocken im Fundus | 5 | 15.3. | 4 | 3 |
| Vereinzelte Plaques im Antrum | 5 | 15.3. | 4 | 3 |
| | 6 | 15.3. | 4 | 3 |

| | Prob. nummer | Datum | Anz. Tabl. | Untersuchungs-zeitpunkt |
|---|---|---|---|---|
| Milchartiger, trueber, flockiger Magensaft im Fundus | 2 | 15.3. | 2 | 2 und 3 |
| | 3 | 15.3. | 2 | 2 |
| | 1 | 15.3. | 4 | 2 |
| | 4 | 15.3. | 4 | 2 und 3 |
| Duennfluessige, milchige Auflagerungen im Fundus | 7 | 15.3. | 2 | 2 |
| | 8 | 15.3. | 2 | 2 und 3 |
| Fundus flaechig, filmartig belegt | 3 | 15.3. | 2 | 3 |
| Antrum und Fundus mit schorfig-milchigem Belag | 5 | 15.3. | 4 | 2 |
| Fundus mit truebem, milchartigem Belag | 6 | 15.3. | 4 | 2 und 3 |
| Sehr viel dickfluessig-milchiges Sekret im Fundus | 2 | 22.3. | 4 | 2 und 3 |
| | 3 | 22.3. | 4 | 2 |
| Sehr viel dickfluessig-milchiges Sekret im Antrum | 3 | 22.3. | 4 | 2 |
| Sehr viel truebes, milchartiges Sekret als filmartiger Ueberzug im Fundus | 8 | 22.3. | 4 | 3 |
| Sehr viel Reste von milchigem Ueberzug und Sekret im Fundus | 3 | 22.3. | 4 | 3 |
| Sehr viel Reste von milchigem Ueberzug und Sekret im Antrum | 3 | 22.3. | 4 | 3 |

Dimethylpolisiloxan wird durch Hydrolyse und Polykondensation von Dichlordimethylsilan und Chlortrimethylsilan erhalten. Die verschiedenen Typen unterscheiden sich durch die nominelle Viskosität, welche durch die Nummer beim Substanznamen ausgedrückt wird.

Der Polymerisationsgrad (n = 20 bis 400) ist so, daß die kinematische Viskosität von 20 bis 1000 $mm^2 \cdot s^{-1}$ (20 bis 1.000 cSt) reicht.

Die Strukturformel von Dimethylpolysiloxan ist:

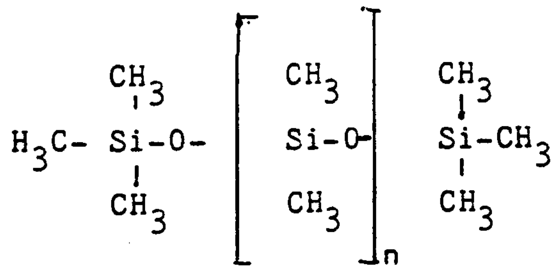

Bei den vorstehend beschriebenen Versuchen wurde Silicon-Antischaummittel S 184 der Firma Wacker Chemie zur Herstellung der Kautabletten verwendet. Gleichwertige Ergebnisse werden auch mit Dimethylpolysiloxan oder Siliconölen mit einer kinematischen Viskosität im Bereich von 100 bis 10000 $mm^2 \cdot s^{-1}$, insbesondere 300 bis 3000 $mm^2 \cdot s^{-1}$ erzielt.

Als hochdisperses Siliciumdioxid wurde Aerosil 2000 eingesetzt.

**Patentansprüche**

1. Verwendung von Dimethylpolysiloxan ohne Zusatz von Antazida zur Herstellung eines Arzneimittels zur Behandlung von ulcerösen Erkrankungen des Magens und Duodenums (Ulcus ventriculi und Ulcus duodeni), entzündlichen Erkrankungen des Ösophagus und der Antrumgastritis.

2. Verwendung nach Anspruch 1 unter Zusatz von Kieselgel.

3. Verwendung nach Anspruch 1 oder 2, wobei das Mittel eine kinematische Viskosität im Bereich von 100 bis 10.000 $mm^2 \cdot s^{-1}$ aufweist.

4. Verwendung nach Anspruch 3, wobei das Mittel eine kinematische Viskosität im Bereich von 300 bis 3.000 $mm^2 \cdot s^{-1}$ aufweist.

5. Verwendung des Mittels nach Anspruch 4, wobei das Mittel eine kinematische Viskosität von etwa 1.000 $mm^2 \cdot s^{-1}$ aufweist.

**Claims**

1. Use of dimethylpolysiloxane without the addition of antacids for the preparation of a medication for the treatment of ulcerous disorders of the stomach and the duodenum (Ulcus ventriculi and Ulcus duodeni), inflammatory disorders of the oesophagus and antral gastritis.

2. Use according to claim 1 with the addition of silica gel.

3. Use of the agent according to claim 1 or 2 with a kinematic viscosity in the range from 100 to 10,000 $mm^2 \cdot s^{-1}$.

4. Use of the agent according to claim 3 with a kinematic viscosity in the range from 300 to 3,000 $mm^2 \cdot s^{-1}$.

5. Use of the agent according to claim 4 with a kinematic viscosity of about 1,000 $mm^2 \cdot s^{-1}$.

**Revendications**

1. Utilisation de diméthylpolysiloxane sans ajout d'anti-acide pour la fabrication d'un agent pharmaceutique en vue du traitement d'affections ulcéreuses de l'estomac et du duodénum (ulcus ventriculi et ulcus duodeni), des affections inflammatoires de l'oesophage et de la gastrite de l'antre.

2. Utilisation selon la revendication 1, avec ajout de silicagel.

**3.** Utilisation selon la revendication 1 ou 2, l'agent présentant une viscosité cinématique dans la plage de 100 à 10 000 $mm^2.s^{-1}$.

**4.** Utilisation selon la revendication 3, l'agent présentant une viscosité cinématique dans la plage de 300 à 3000 $mm^2.s^{-1}$.

**5.** Utilisation de l'agent selon la revendication 4, l'agent présentant une viscosité cinématique d'environ 1000 $mm^2.s^{-1}$.